Europäisches Patentamt

European Patent Office

Office européen des brevets

(11) Veröffentlichungsnummer: **0 602 552 A1**

## EUROPÄISCHE PATENTANMELDUNG

(21) Anmeldenummer: **93119888.1**

(51) Int. Cl.5: **C07C 211/52**

(22) Anmeldetag: **09.12.93**

(30) Priorität: **18.12.92 DE 4242897**

(43) Veröffentlichungstag der Anmeldung:
**22.06.94 Patentblatt 94/25**

(84) Benannte Vertragsstaaten:
**BE CH DE ES FR GB IT LI NL**

(71) Anmelder: **HOECHST AKTIENGESELLSCHAFT**
**Brüningstrasse 50**
**D-65929 Frankfurt am Main(DE)**

(72) Erfinder: **Papenfuhs, Theodor Dr.**
**Heinrich-Bleicher-Strasse 40**
**D-60433 Frankfurt am Main(DE)**
Erfinder: **Planker, Siegfried Dr.**
**Kastanienweg 17**
**D-61462 Königstein/Ts(DE)**

(54) **2,3,4-Trifluor-N-ethylanilin und Verfahren zu seiner Herstellung.**

(57) Die vorliegende Erfindung betrifft die Verbindung 2,3,4-Trifluor-N-ethylanilin und ein Verfahren zu seiner Herstellung.

Man stellt 2,3,4-Trifluor-N-ethylanilin her, indem man 1 mol 2,3,4-Trifluornitrobenzol in einem gegenüber den Reaktionsteilnehmern und bei den Reaktionsbedingungen inerten organischen Lösungsmittel als Verdünnungsmittel in Gegenwart eines modifizierten Nickel- oder Edelmetallkatalysators und einer Puffersubstanz bei Temperaturen von etwa 80°C bis etwa 120°C bei pH-Werten von etwa 7 bis 10 bei Atmosphärendruck oder Überdruck mit Wasserstoff zum 2,3,4-Trifluoranilin reduziert und dieses anschließend durch Zugabe von 1 bis etwa 1,5 mol Acetaldehyd, bezogen auf das gebildete 2,3,4-Trifluoranilin, gegebenenfalls zusammen mit einem inerten organischen Lösungsmittel als Verdünngsmittel, bei Temperaturen von etwa 40°C bis etwa 100°C mit Wasserstoff alkylierend reduziert.

EP 0 602 552 A1

Die Erfindung betrifft das neue 2,3,4-Trifluor-N-ethylanilin und Verfahren zu seiner Herstellung durch katalytische Hydrierung von 2,3,4-Trifluornitrobenzol zum 2,3,4-Trifluoranilin und dessen alkylierende katalytische Hydrierung in Gegenwart von Acetaldehyd.

Das 2,3,4-Trifluor-N-ethylanilin stellt ein wertvolles Vorprodukt zur Herstellung von Pharmazeutika und Pflanzenschutzmitteln dar.

Es ist prinzipiell bekannt, daß N-Alkyl-arylamine durch reduktive Alkylierung aromatischer Nitroverbindungen bzw. Anilinderivate durch katalytische Hydrierung in Gegenwart aliphatischer Aldehyde und Ketone hergestellt werden können (DE 3 433 924; EP 04 11 817; Hydrogenation Methods, P. N. Rylander, Seite 82 bis 91).

Es wurde nun gefunden, daß man 2,3,4-Trifluor-N-ethylanilin in befriedigender Ausbeute in einem "Ein-Topf-Verfahren" herstellen kann, indem man 1 mol 2,3,4-Trifluornitrobenzol in einem gegenüber den Reaktionsteilnehmern und bei den Reaktionsbedingungen inerten organischen Lösungsmittel als Verdünnungsmittel in Gegenwart eines modifizierten Nickel- oder Edelmetallkatalysators bei Temperaturen von etwa 80°C bis etwa 120°C, vorzugsweise etwa 85°C bis etwa 95°C, bei pH-Werten von etwa 7 bis etwa 10 bei Atmosphärendruck oder Überdruck mit Wasserstoff zum 2,3,4-Trifluoranilin reduziert und dieses anschließend durch Zugabe von 0,8 bis etwa 1,5 mol, vorzugsweise 1,0 bis etwa 1,2 mol Acetaldehyd, bezogen auf das gebildete 2,3,4-Trifluoranilin, gegebenenfalls zusammen mit einem inerten organischen Lösungsmittel als Verdünnungsmittel, bei Temperaturen von etwa 40°C bis etwa 100°C, vorzugsweise etwa 60°C bis etwa 80°C mit Wasserstoff alkylierend reduziert.

Die Eignung der verfahrensgemäß eingesetzten Katalysatoren hängt vom Grad ihrer Selektivität ab. Die Anwendung von bei katalytischen Hydrierungen üblicherweise eingesetzten Nickel- oder Edelmetallkatalysatoren in modifizierter Form ist deshalb geboten, weil die Reduktion (Hydrierung) mit den nicht modifizierten Nickel- oder Edelmetallkatalysatoren wegen der als Nebenreaktion auftretenden Abspaltung von Fluorwasserstoff auch bei Wahl milder Reaktionsbedingungen nur unbefriedigend durchführbar ist. Bevorzugt werden verfahrensgemäß sulfitierte oder sulfidierte Platin- oder Palladiumkatalysatoren, besonders bevorzugt in Form von Trägerkatalysatoren, verwendet. Das Trägermaterial, auf welchem die Katalysatormetalle fein verteilt sind, besteht beispielsweise aus Aktivkohle, Aluminiumoxid, Bariumsulfat, Bimsstein, Tonerde, Kieselgur oder Kieselgel. Besonders wirksam sind Platin- oder Palladium-Trägerkatalysatoren, deren Trägermaterial aus Aktivkohle oder Aluminiumoxid besteht.

Die anwendbaren Nickel- oder Edelmetallkatalysatoren können zusätzlich mit Schwefelverbindungen, wie beispielsweise Sulfoxiden, insbesondere mit Dimethylsulfoxid, modifiziert sein.

Durch Zusatz von Puffersubstanzen kann eine Steigerung der selektiven Wirkung der Katalysatoren bewirkt werden. Geeignete Puffersubstanzen sind beispielsweise Dinatriumhydrogenphosphat, Natriumacetat, primäre, sekundäre oder tertiäre aliphatische Amine der allgemeinen Formel

$$NH_xR_y$$

in welcher R einen geradkettigen oder verzweigten Alkylrest-$C_nH_{2n+1}$, worin n für eine Zahl von 1 bis etwa 6 steht, x = 0,1 oder 2, y = 1, 2 oder 3 und x + y = 3 bedeuten, wie beispielsweise Methylamin, Diäthylamin oder Triethylamin, oder cyclische Amine, wie beispielsweise Morpholin oder N-Methylmorpholin. Anstelle von Puffersalzen können auch verdünnte wäßrige Alkalimetall- oder Erdalkalimetallhydroxidlösungen, wie beispielsweise verdünnte wäßrige Natrium- oder Kaliumhydroxidlösungen, verwendet werden.

Die verfahrensgemäß bevorzugt eingesetzten modifizierten Platin- oder Palladium-Trägerkatalysatoren enthalten zweckmäßigerweise etwa 0,1 bis etwa 10 Gew.-%, vorzugsweise etwa 1 bis etwa 5 Gew.-% Platin oder Palladium, bezogen auf den gesamten Katalysator.

Es ist ferner zweckmäßig, etwa 5 bis etwa 200 mg Platin oder Palladium pro mol 2,3,4-Trifluornitrobenzol anzuwenden.

Die gegebenenfalls als Verdünnungsmittel angewandten inerten organischen Lösungsmittel sind beispielsweise niedere aliphatische Alkohole, wie beispielsweise Methanol, Ethanol oder i-Propanol, ferner aromatische Kohlenwasserstoffe, wie beispielsweise Toluol oder Xylole, ferner Essigsäurealkyl($C_1$-$C_6$)-ester, wie beispielsweise Ethylacetat und Butylacetat.

Das Verfahren wird im allgemeinen im Druckbereich von 1 bis etwa 40 bar, vorzugsweise etwa 5 bis etwa 20 bar durchgeführt. Setzt man bei Überdruck um, so wird im Autoklav gearbeitet.

Eine zweckmäßige Ausführung des erfindungsgemäßen Verfahrens besteht darin, daß man das 2,3,4-Trifluornitrobenzol, gegebenenfalls mit einem inerten organischen Lösungsmittel als Verdünnungsmittel, zusammen mit dem Katalysator und der gegebenenfalls verwendeten Puffersubstanz in einem Autoklav vorlegt, nach erfolgter katalytischer Hydrierung des 2,3,4-Trifluornitrobenzols mit Wasserstoff zum 2,3,4-Trifluoranilin den Acetaldehyd, gegebenenfalls mit einem inerten organischen Lösungsmittel (Verdünnungsmittel), zupumpt und die alkylierende Reduktion zu Ende führt. Anschließend wird der Katalysator durch Filtrieren abgetrennt.

Die zurückgebliebene Lösung wird durch Destillation aufgearbeitet.

Das 2,3,4-Trifluor-N-ethylanilin kann auch hergestellt werden, in dem man direkt vom 2,3,4-Trifluoranilin ausgeht. In diesem Fall legt man zweckmäßigerweise 1 mol 2,3,4-Trifluoranilin, gegebenenfalls zusammen mit einem inerten organischen Lösungsmittel (Verdünnungsmittel) mit dem sulfitierten oder sulfidierten Platin- oder Palladium-Trägerkatalysator und der Puffersubstanz im Reaktionsgefäß vor, führt der vorgelegten Mischung bei Temperaturen von etwa 40°C bis etwa 100°C, vorzugsweise etwa 60°C bis etwa 80°C gleichzeitig 0,8 bis etwa 1,5 mol, vorzugsweise 1 bis etwa 1,2 mol Acetaldehyd, gegebenenfalls zusammen mit einem inerten organischen Lösungsmittel (Verdünnungsmittel), und Wasserstoff zu und reduziert alkylierend bis zur Vollständigkeit.

Die nachstehenden Beispiele dienen zur Erläuterung des erfindungsgemäßen Verfahrens, ohne es darauf zu beschränken.

Beispiel 1

In einem Stahlautoklav, ausgerüstet mit Magnethubrührer, Heizvorrichtung und Kühlung, werden 267 g (1,5 mol) 2,3,4-Trifluornitrobenzol, 435 g (550 ml) Methanol, 2 g (trocken) bzw. 4 g (feucht) 5 %iger Platin (auf Kohlenstoff)-Katalysator (sulfitiert) und 2 g Morpholin vorgelegt. Nach Verdrängen der Luft im verschlossenen Autoklav mit Stickstoff und nach erfolgtem Austausch des Stickstoffs durch Wasserstoff wird das Reaktionsgemisch unter Rühren auf 85 bis 95°C erhitzt und bei dieser Temperatur und einem Wasserstoffdruck von 5 bis 15 bar hydriert. Nach beendeter Hydrierung (unter Bildung des 2,3,4-Trifluoranilins) wird die Reaktionsmischung auf etwa 60°C abgekühlt.

Anschließend werden 80 g (1,8 mol) Acetaldehyd, gelöst in 79 g (100 ml) Methanol, innerhalb 1 Stunde zugepumpt. Die Reduktion der als Zwischenstufe gebildeten Schiff'schen Base setzt sofort ein. Durch Aufdrücken von Wasserstoff wird der Druck zwischen 15 und 20 bar gehalten. Wenn kein Druckabfall mehr erfolgt, rührt man noch 30 Minuten bei 60°C bis 80°C und 20 bar Wasserstoffdruck nach.

Nach Austausch des Wasserstoffs im Autoklav durch Stickstoff und Entspannen wird der Katalysator bei 40°C bis 60°C über ein Druckfilter unter Stickstoff abgetrennt und in den Folgeansatz zurückgeführt.

Aus dem Filtrat wird das Lösungsmittel (Methanol) abdestilliert und das Produkt anschließend durch fraktionierte Destillation im Vakuum von Nebenprodukten abgetrennt.
Ausbeute: 183 g, entsprechend 70 d. Th.
Siedepunkt: 122°C bei 60 mm Hg; RG und GC: ≧ 98 %

Beispiel 2

In einem Stahlautoklav, ausgerüstet mit Magnethubrührer, Heizvorrichtung und Kühlung, werden 220,5 g (1,5 mol) 2,3,4-Trifluoranilin, 435 g (550 ml) Methanol, 2 g (trocken) bzw. 4 g (feucht) 5 %iger Platin (auf Kohlenstoff)-Katalysator (sulfitiert) und 2 g Triethylamin vorgelegt. Nach Verdrängen der Luft im verschlossenen Autoklav mit Stickstoff und nach erfolgtem Austausch des Stickstoffs durch Wasserstoff wird das Reaktionsgemisch auf 60°C erhitzt und unter Rühren Wasserstoff bis auf 20 bar aufgedrückt. Gleichzeitig werden 80 g (1,8 mol) Acetaldehyd, gelöst in 79 g (100 ml) Methanol, innerhalb 1 Stunde zugepumpt. Die Reduktion der als Zwischenstufe gebildeten Schiff'schen Base setzt sofort ein. Durch Aufdrücken von Wasserstoff wird der Druck zwischen 15 und 20 bar gehalten. Wenn kein Druckabfall mehr erfolgt, rührt man noch 30 Minuten bei 60°C bis 80°C und 20 bar Wasserstoffdruck nach.

Nach Austausch des Wasserstoffs im Autoklav durch Stickstoff und Entspannen wird der Katalysator bei 40°C bis 60°C über ein Druckfilter unter Stickstoff abgetrennt und in den Folgeansatz zurückgeführt.

Aus dem Filtrat wird das Lösungsmittel (Methanol) abdestilliert und das Produkt anschließend durch fraktionierte Destillation im Vakuum von Nebenprodukten abgetrennt.
Ausbeute: 197 g, entsprechend 75 % d. Th.
Siedepunkt: 122°C bei 60 mm Hg; RG und GC: ≧ 98 %

Verwendet man anstelle des sulfitierten Platin (auf Kohlenstoff)-Katalysators die gleiche Menge an sulfidiertem Platin (auf Kohlenstoff)-Katalysator und arbeitet im übrigen wie in Beispiel 2 angegeben, so gelangt man praktisch zum gleichen Ergebnis.

2,3,4-Trifluor-N-ethylanilin ist durch folgende kernmagnetische Resonanz-Daten charakterisiert:

$^1$H-NMR [CDCl$_3$, TMS, ppm]:

$\delta$ = 1.28 (tr, 3H, $J_{DE}$ = 7.08 Hz, -CH$_3$ (E))

3.15 (qu, 2H, $J_{DE}$ = 7.08 Hz, -CH$_2$-(D))

3.66 (s(br), 1H, -NH-(C))

6.32 (dddd, 1H, $J_{AB}$ = 9.22 Hz, $J_{BX}$ = 4.65 Hz, $J_{BY}$ = 8.81 Hz, $J_{BZ}$ = 2.45 Hz, Ar-H$^6$ (B))

6.80 (dddd, 1H, $J_{AB}$ = 9.22 Hz, $J_{AX}$ = 10.06 Hz, $J_{AY}$ = 2.48 Hz, $J_{AZ}$ = 7.99 Hz, Ar-H$^5$ (A))

$^{19}$F-NMR [CDCl$_3$, CFCl$_3$, ppm]:

$\delta$ = -152.12(dddd, 1F, $J_{AX}$ = 10.06 Hz, $J_{XY}$ = 1.37 Hz, $J_{XZ}$ = 21.22 Hz, $J_{BX}$ = 4.65 Hz, Ar-F$^4$ (X))

-158.66 (dddd, 1F, $J_{AY}$ = 2.48 Hz, $J_{XY}$ = 1.37 Hz, $J_{YZ}$ = 19.37 Hz, $J_{BY}$ = 8.81 Hz, Ar-F$^2$ (Y))

-162.25 (dddd, 1F, $J_{AZ}$ = 7.99 Hz, $J_{BZ}$ = 2.45 Hz, $J_{XZ}$ = 21.22 Hz, $J_{YZ}$ = 19.37 Hz, Ar-F$^3$ (Z))

**Patentansprüche**

1.   2,3,4-Trifluor-N-ethylanilin

2.   Verfahren zur Herstellung von 2,3,4-Trifluor-N-ethylanilin, dadurch gekennzeichnet, daß man 1 mol 2,3,4-Trifluornitrobenzol in einem gegenüber den Reaktionsteilnehmern und bei den Reaktionsbedingungen inerten organischen Lösungsmittel als Verdünnungsmittel in Gegenwart eines modifizierten Nickel- oder Edelmetallkatalysators und einer Puffersubstanz bei Temperaturen von etwa 80°C bis etwa 120°C bei pH-Werten von etwa 7 bis etwa 10 bei Atmosphärendruck oder Überdruck mit Wasserstoff zum 2,3,4-Trifluoranilin reduziert und dieses anschließend durch Zugabe von 1 bis etwa 1,5 mol Acetaldehyd, bezogen auf das gebildete 2,3,4-Trifluoranilin, gegebenenfalls zusammen mit einem inerten organischen Lösungsmittel als Verdünnungsmittel, bei Temperaturen von etwa 40°C bis etwa 100°C mit Wasserstoff alkylierend reduziert.

3.   Verfahren nach Anspruch 2, dadurch gekennzeichnet, daß man die Umsetzung des 2,3,4-Trifluoranilin zum 2,3,4-Trifluor-N-ethylanilin bei Temperaturen von etwa 60°C bis etwa 80°C vornimmt.

4.   Verfahren nach mindestens einem der Ansprüche 2 und 3, dadurch gekennzeichnet, daß man in Gegenwart eines sulfitierten oder sulfidierten Edelmetall-Trägerkatalysators reduziert.

5.   Verfahren nach mindestens einem der Ansprüche 2 bis 4, dadurch gekennzeichnet, daß das Trägermaterial aus Aktivkohle, Aluminiumoxid, Bariumsulfat, Bimsstein, Tonerde, Kieselgur oder Kieselgel besteht.

6.   Verfahren nach mindestens einem der Ansprüche 2 bis 5, dadurch gekennzeichnet, daß man in Gegenwart eines sulfitierten oder sulfidierten Platin- oder Palladium-Trägerkatalysators reduziert, dessen Trägermaterial aus Aktivkohle oder Aluminiumoxid besteht.

7.   Verfahren nach mindestens einem der Ansprüche 2 bis 6, dadurch gekennzeichnet, daß der Platin- oder Palladium-Trägerkatalysator etwa 0,1 bis etwa 10 Gew.-% Platin oder Palladium, bezogen auf den gesamten Katalysator, enthält.

8.   Verfahren nach mindestens einem der Ansprüche 2 bis 7, dadurch gekennzeichnet, daß der Platin- oder Palladium-Trägerkatalysator etwa 1 bis etwa 5 Gew.-% Platin oder Palladium, bezogen auf den gesamten Katalysator, enthält.

9.   Verfahren nach mindestens einem der Ansprüche 2 bis 8, dadurch gekennzeichnet, daß man etwa 5 bis etwa 200 mg Platin oder Palladium pro mol 2,3,4-Trifluornitrobenzol anwendet.

10.  Verfahren nach mindestens einem der Ansprüche 2 bis 9, dadurch gekennzeichnet, daß man als inerte organische Lösungsmittel Methanol, Ethanol, i-Propanol, Toluol, Xylole oder Essigsäurealkyl($C_1$-$C_6$)ester verwendet.

11.  Verfahren nach mindestens einem der Ansprüche 2 bis 10, dadurch gekennzeichnet, daß man als inerte organische Lösungsmittel Ethylacetat oder n-Butylacetat verwendet.

12.  Verfahren nach mindestens einem der Ansprüche 2 bis 11, dadurch gekennzeichnet, daß man in Gegenwart von Dinatriumhydrogenphosphat, Natriumacetat, primären, sekundären oder tertiären aliphatischen Aminen der allgemeinen Formel

$NH_xR_y$

in welcher R einen geradkettigen oder verzweigten Alkylrest-$C_nH_{2n+1}$, worin n für eine Zahl von 1 bis etwa 6 steht, $x = 0,1$ oder 2, $y = 1$, 2 oder 3 und $x + y = 3$ bedeuten, umsetzt.

13.  Verfahren nach mindestens einem der Ansprüche 2 bis 12, dadurch gekennzeichnet, daß man in Gegenwart von Methylamin, Diäthylamin, Triethylamin oder Morpholin als Puffersubstanzen umsetzt.

14.  Verfahren nach mindestens einem der Ansprüche 2 bis 13, dadurch gekennzeichnet, daß man 0,8 bis etwa 1,2 mol Acetaldehyd, bezogen auf 1 mol 2,3,4-Trifluoranilin, einsetzt.

15.  Verfahren nach mindestens einem der Ansprüche 2 bis 14, dadurch gekennzeichnet, daß man bei einem Druck von etwa 1 bis etwa 40 bar arbeitet.

16.  Verfahren nach mindestens einem der Ansprüche 2 bis 15, dadurch gekennzeichnet, daß man bei einem Druck von etwa 5 bis etwa 20 bar arbeitet.

17.  Verfahren nach mindestens einem der Ansprüche 2 bis 16, dadurch gekennzeichnet, daß man das 2,3,4-Trifluornitrobenzol, gegebenenfalls zusammen mit dem inerten organischen Lösungsmittel (Verdünnungsmittel), mit dem Katalysator und der Puffersubstanz in einem Autoklav vorlegt, nach erfolgter Bildung des 2,3,4-Trifluoranilins den Acetaldehyd, gegebenenfalls mit einem inerten organischen Lösungsmittel (Verdünnungsmittel), zupumpt und die alkylierende Reduktion zu Ende führt.

18. Verfahren zur Herstellung von 2,3,4-Trifluor-N-ethylanilin, dadurch gekennzeichnet, daß man 1 mol 2,3,4-Trifluoranilin, gegebenenfalls zusammen mit einem inerten organischen Lösungsmittel (Verdünnungsmittel), mit dem sulfitierten oder sulfidierten Platin-oder Palladium-Trägerkatalysator und der Puffersubstanz im Reaktionsgefäß vorlegt, dieser vorgelegten Mischung bei Temperaturen von etwa 40°C bis etwa 100°C gleichzeitig 0,8 bis etwa 1,5 mol Acetaldehyd, gegebenenfalls zusammen mit einem inerten organischen Lösungsmittel (Verdünnungsmittel), und Wasserstoff zuführt und erschöpfend alkylierend reduziert.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A,D | EP-A-0 411 817 (SUMITOMO CHEMICAL COMPANY LIMITED) <br> * Ansprüche * <br><br> --- | 2-18 | C07C211/52 |
| A | EP-A-0 318 893 (CIBA-GEIGY AG) <br> * Seite 16 * <br><br> ----- | 1-18 | |

| RECHERCHIERTE SACHGEBIETE (Int.Cl.5) |
|---|
| C07C |

Der vorliegende Recherchenbericht wurde für alle Patentansprüche erstellt

| Recherchenort | Abschlußdatum der Recherche | Prüfer |
|---|---|---|
| DEN HAAG | 30. März 1994 | Sanchez y Garcia, J |